# EUROPEAN PATENT APPLICATION

(11) **EP 2 436 643 A1**
(43) Date of publication of application: **04.04.2012**
(21) Application number: 11183363.8
(22) Date of filing: 29.09.2011
(51) Int. Cl.: C01B 3/06, A61M 16/00

(54) **Hydrogen production device, hydrogen addition device and hydrogen-additive article**

(30) Priority: 30.09.2010 TW 099133194
(71) Applicant: Young Green Energy Co., Hsinchu County 30352 (TW)
(72) Inventor: Huang, Shao-Chi, 30352 Hukou Township, Hsinchu County (TW); Hung, Chieh-Ju, 30352 Hukou Township, Hsinchu County (TW); Wang, Cheng, 30352 Hukou Township, Hsinchu County (TW); Chou, Po-Kuei, 30352 Hukou Township, Hsinchu County (TW); Cheng, Tsai-Hsin, 30352 Hukou Township, Hsinchu County (TW)
(74) Representative: TER MEER - STEINMEISTER & PARTNER GbR

(57) **Abstract**

A hydrogen production device includes a reaction unit, a hydrogen collection chamber, and a flow channel. The reaction unit stores a first reactant and a second reactant. When the first reactant comes into contact the second reactant, the first reactant reacts with the second reactant to produce hydrogen gas. The hydrogen collection chamber has at least one gas vent to lead the hydrogen gas to flow into a hydrogen acceptor via the gas vent. The flow channel is in communication with the reaction unit and the hydrogen collection chamber to enable the hydrogen gas to flow into the hydrogen collection chamber.

## Description

### BACKGROUND OF THE INVENTION

### a. Field of the Invention

The invention relates to a hydrogen production device, a hydrogen addition device, and a hydrogen-additive article produced by the hydrogen addition device.

### b. Description of the Related Art

Nowadays, hydrogen gas (H₂) has been extensively utilized in prophylaxis and therapy. For example, hydrogen gas may quickly diffuse into and permeate a cell membrane to effectively reduce toxic radicals to protect cells from being oxidized and destroyed. Therefore, hydrogen gas often serves as an anti-oxidant having a curative effect and can protect DNA (deoxyribonucleic acid) in a cell nucleus so as to reduce the risk of suffering from cancers. Besides, hydrogen gas does not disturb redox reactions or cellular signal transduction and may directly prevent cell destruction caused by strong oxidizing active substance in living cells. For example, inflammation, severe excise, myocardial infarction, blood vessel blockage, organ transplant, etc. may result in acute oxidized stimulation. Further, hydrogen gas may dissolve in water and enter a human body through drinking to carry away radicals. Alternatively, one may directly inhale hydrogen gas. Generally, the concentration of hydrogen gas less than 4.7% will not cause ignition and explosion, and thus hydrogen gas could extensively serve as a safe and effective anti-oxidant with a minimal side effect in treatment and cosmetology.

However, since it is inconvenient to carry and store hydrogen gas, commercial ready-to-serve hydrogen-additive articles for the clinic and cosmetic purposes are still not available. US patent publication no. 2009/0004512, Taiwan patent publication no. 201022138, Taiwan patent no. I328620, and Taiwan patent no. I301158 all disclose the production of hydrogen gas where solid-state reacting particles react with a liquid to produce hydrogen. Taiwan patent publication no. 200703763 discloses a fuel container having a solid-state fuel and a liquid-state fuel separated by a membrane. Taiwan patent no. M358828 and Taiwan patent no. M330576 disclose a design of transporting hydrogen gas to the external environment through a hydrogen output pipeline. Further, US patent publication no. 2005/0224996 discloses a design of discharging produced hydrogen gas into water. However, according to the above designs, it is difficult to produce portable and ready-to-serve hydrogen gas products, and it is also difficult to add hydrogen gas into an article.

### BRIEF SUMMARY OF THE INVENTION

The invention provides a hydrogen production device and a hydrogen addition device. The hydrogen production device and the hydrogen addition device have at least one of the advantages of excellent portability, ready-to-use, and hydrogen gas being easily added into an article.

The invention also provides a hydrogen-additive article. Hydrogen gas is easily added into the hydrogen-additive article through a simple operation, and the hydrogen-additive article is taken or used by a person immediately after the hydrogen gas is produced.

Other objects and advantages of the invention can be better understood from the technical characteristics disclosed by the invention.

In order to achieve one of the above purposes, all the purposes, or other purposes, one embodiment of the invention provides a hydrogen production device, including a reaction unit, a hydrogen collection chamber, and a flow channel. The reaction unit stores a first reactant and a second reactant. When the first reactant comes into contact the second reactant, the first reactant reacts with the second reactant to produce hydrogen gas. The hydrogen collection chamber has at least one gas vent to lead the hydrogen gas to flow into a hydrogen acceptor via the gas vent. The flow channel is in communication with the reaction unit and the hydrogen collection chamber to enable the hydrogen gas to flow into the hydrogen collection chamber.

In one embodiment, the first reactant is a solid-state reactant and the second reactant is a liquid-state reactant. Alternatively, the first reactant is a liquid-state reactant and the second reactant is a solid-state reactant.

In one embodiment, the hydrogen production device further includes a barrier layer for separating the first reactant from the second reactant.

In one embodiment, the hydrogen collection chamber further includes a filtering region to purify the hydrogen gas.

In one embodiment, the hydrogen collection chamber further includes an odor-adding region.

In one embodiment, the flow channel includes a water-repellent floating ball and a water-repellent hydrogen conduit. One end of the water-repellent hydrogen conduit is connected to the water-repellent floating ball, the other end of the water-repellent hydrogen conduit is connected to the hydrogen collection chamber, and the produced hydrogen gas flows into the water-repellent floating ball and the water-repellent hydrogen conduit by a pressure difference.

In one embodiment, the hydrogen collection chamber includes a fluid region.

In one embodiment, the hydrogen production device further includes an observation window used to confirm whether a hydrogen production reaction occurs, and the observation window includes at least one check valve.

In one embodiment, at least one through-hole formed on a side wall of the hydrogen production device to form an air flow channel in communication with an external environment.

In one embodiment, the hydrogen collection chamber further includes an air-mixing region to adjust the concentration of the hydrogen gas flowing into the hydrogen acceptor.

In one embodiment, a concentration of hydrogen gas in the mixture is in a range of 0.1%-10% and a cross-sectional area of the gas vent is equal to about 2% of a cross-sectional area of the air-mixing region.

In one embodiment, the hydrogen production device further includes a suction device in communication with the gas vent. At least one through-hole is formed on a side wall of the suction device to form an air flow channel in communication with an external environment.

In one embodiment, the hydrogen acceptor is a human body, a drink, or cosmetics.

According to another embodiment of the invention, a hydrogen addition device includes a reaction unit and a penetration element. The reaction unit includes a first reactant and a second reactant, and the first reactant comes into contact and reacts with the second reactant to produce hydrogen gas. The penetration element is disposed between the reaction unit and a hydrogen acceptor, and the penetration element enables the reaction unit and the hydrogen acceptor to change from an isolation state to a communication state. The hydrogen acceptor may be a drink or cosmetics.

In one embodiment, the penetration element penetrates or pushes a part of a side wall of the hydrogen acceptor to allow the produced hydrogen to dissolve in the hydrogen acceptor.

In one embodiment, the penetration element has a helical ridge structure with a sharp end, the hydrogen acceptor has a thread structure complementary to the helical ridge structure in shape, the helical ridge structure is allowed to pierce a part of a side wall of the hydrogen acceptor, and the helical ridge structure is tightly fit with the thread structure.

Another embodiment of the invention provides a hydrogen-additive article. The hydrogen-additive article contains hydrogen gas supplied by a hydrogen production device. The hydrogen production device at least includes a reaction unit, a hydrogen collection chamber and a flow channel. The flow channel is in communication with the reaction unit and the hydrogen collection chamber to enable a hydrogen gas produced in the reaction unit to flow into the hydrogen collection chamber, and the hydrogen collection chamber has at least one gas vent to lead the hydrogen gas into the hydrogen-additive article.

In conclusion, the embodiment or the embodiments of the invention may have at least one of the following advantages.

According to the above embodiments, a person may directly inhale hydrogen gas just after the hydrogen gas is produced through a very simple operation, or the produced hydrogen gas is added into a hydrogen acceptor (such as a drink or cosmetics) and immediately taken or used by a person, with the concentration of the hydrogen gas being instantaneously adjusted in different circumstances. Accordingly, compared with conventional designs, the hydrogen production device according to the above embodiments is more portable, ready-to-serve, easy to add hydrogen gas into an article, and without the need of electronic devices or expensive components. Therefore, this also simplifies fabrication processes and lowers fabrication costs.

Other objectives, features and advantages of the invention will be further understood from the further technological features disclosed by the embodiments of the invention wherein there are shown and described preferred embodiments of this invention, simply by way of illustration of modes best suited to carry out the invention.

### BRIEF DESCRIPTION OF THE DRAWINGS

FIG. 1 shows a schematic diagram of a hydrogen production device according to an embodiment of the invention.

FIG. 2 shows a schematic diagram illustrating hydrogen production processes according to an embodiment of the invention.

FIG. 3 shows a schematic diagram of a hydrogen production device according to another embodiment of the invention.

FIG. 4A shows a schematic diagram illustrating an air-mixing method according to an embodiment of the invention.

FIG. 4B shows a schematic diagram illustrating an air-mixing method according to another embodiment of the invention.

FIG. 5 shows a schematic diagram illustrating an internal structure of a hydrogen production device according to an embodiment of the invention.

FIG. 6 shows a schematic diagram illustrating the appearance of the hydrogen production device shown in FIG. 5.

FIGS. 7A and 7B show schematic diagrams of an observation window according to an embodiment of the invention.

FIGS. 8A and 8B show schematic diagrams of an air-mixing mechanism according to an embodiment of the invention.

FIG. 9 shows a schematic diagram of an air-mixing mechanism according to another embodiment of the invention.

FIG. 10 shows a schematic diagram of a hydrogen addition device according to an embodiment of the invention.

FIG. 11 shows a schematic diagram of a hydrogen addition device according to another embodiment of the invention.

### DETAILED DESCRIPTION OF THE INVENTION

In the following detailed description of the preferred embodiments, reference is made to the accompanying drawings which form a part hereof, and in which are shown by way of illustration specific embodiments in which the invention may be practiced. In this regard, directional terminology, such as "top," "bottom," "front," "back," etc., is used with reference to the orientation of the Figure(s) being described. The components of the invention can be positioned in a number of different orientations. As such, the directional terminology is used for purposes of illustration and is in no way limiting. On the other hand, the drawings are only schematic and the sizes of components may be exaggerated for clarity. It is to be understood that other embodiments may be utilized and structural changes may be made without departing from the scope of the invention. Also, it is to be understood that the phraseology and terminology used herein are for the purpose of description and should not be regarded as limiting. The use of "including," "comprising," or "having" and variations thereof herein is meant to encompass the items listed thereafter and equivalents thereof as well as additional items. Unless limited otherwise, the terms "connected," "coupled," and "mounted" and variations thereof herein are used broadly and encompass direct and indirect connections, couplings, and mountings. Similarly, the terms "facing," "faces" and variations thereof herein are used broadly and encompass direct and indirect facing, and "adjacent to" and variations thereof herein are used broadly and encompass directly and indirectly "adjacent to". Therefore, the description of "A" component facing "B" component herein may contain the situations that "A" component directly faces "B" component or one or more additional components are between "A" component and "B" component. Also, the description of "A" component "adjacent to" "B" component herein may contain the situations that "A" component is directly "adjacent to" "B" component or one or more additional components are between "A" component and "B" component. Accordingly, the drawings and descriptions will be regarded as illustrative in nature and not as restrictive.

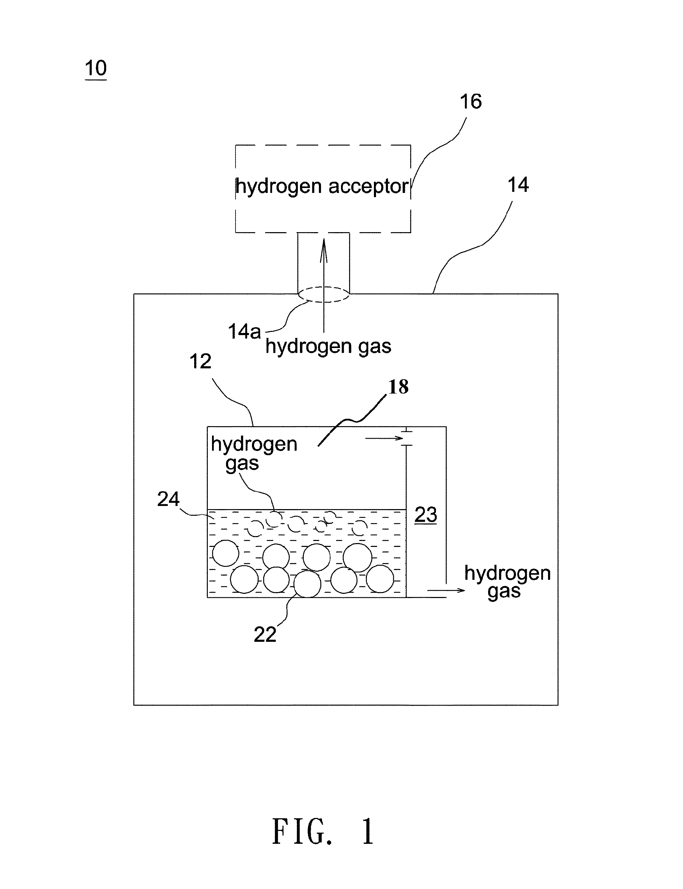

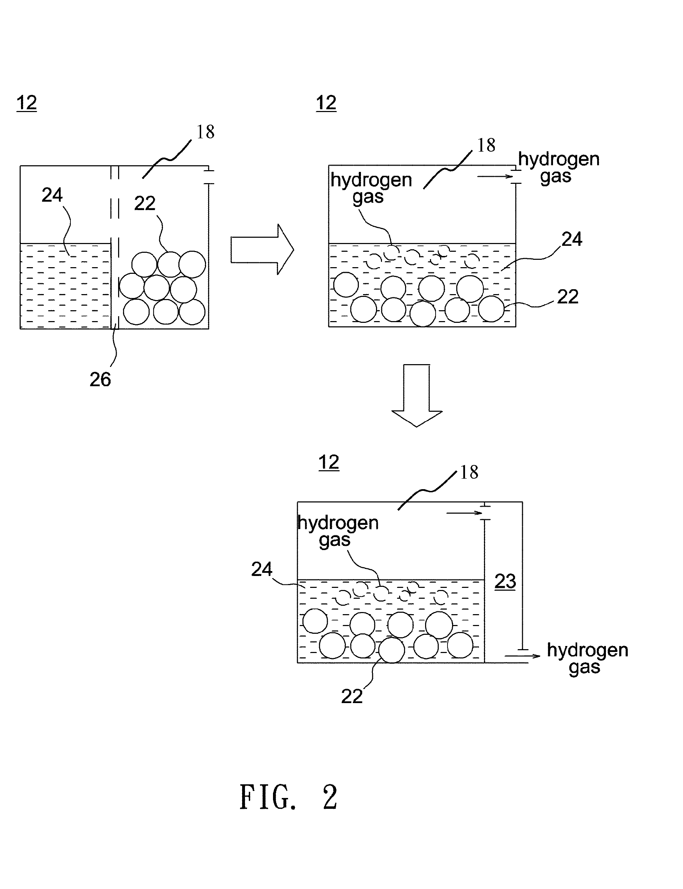

As shown in FIG. 1, a hydrogen production device 10 includes a reaction unit 12, a hydrogen collection chamber 14, and a flow channel 18. A first reactant 22, for example, a solid-state reactant, and a second reactant 24, for example, a liquid-state reactant, are stored in the reaction unit 12. When the first reactant 22 comes into contact with the second reactant 24, they react with each other to produce hydrogen gas. The flow channel 18 is in communication with the hydrogen chamber 14 and the reaction unit 12, the hydrogen gas is allowed to flow into the hydrogen collection unit 14 through the flow channel 18, and the second reactant 24 is prevented from entering the hydrogen collection chamber 14. The hydrogen gas flows into a hydrogen acceptor 16 via a gas vent 14a of the hydrogen collection chamber 14. The hydrogen acceptor 16 may be, for example, a human body, a drink, cosmetics, etc. Referring to FIG. 2, in the reaction unit 12, a barrier layer 26 (such as a diaphragm) separates the first reactant 22 in the first region from the second reactant 24 in the second region. When the barrier layer 26 is pressurized to form an opening, the first reactant 22 comes into contact and reacts with the second reactant 24 to produce hydrogen gas. The produced hydrogen gas passes at least one filtering region 23 to remove moisture and impurities. Additionally, the compositions of the first reactant 22 and the second reactant 24 are not limited, as long as the reaction for producing hydrogen gas occurs. In one embodiment, the first reactant could be a liquid-state reactant and the second reactant could be a solid-state reactant. Otherwise, the solid-state reactant includes, for example, Sodium Borohydride, Magnesium Hydride, Aluminum powders, etc. and the liquid-state reactant includes, for example, water, Cobaltous Chloride (CoCl₂), Nickelous Chloride (NiCl₂), etc.

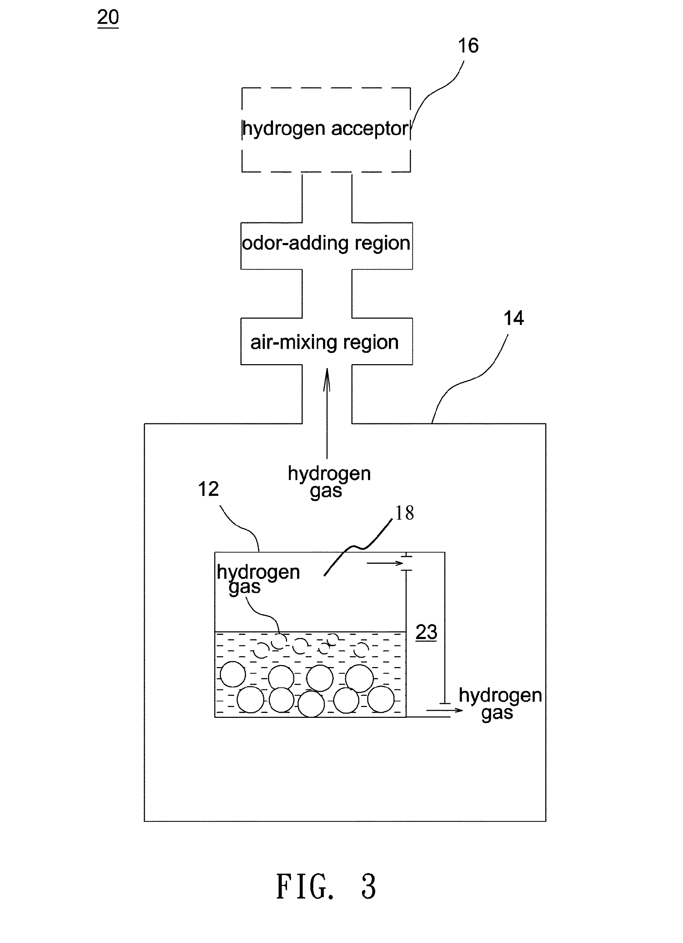

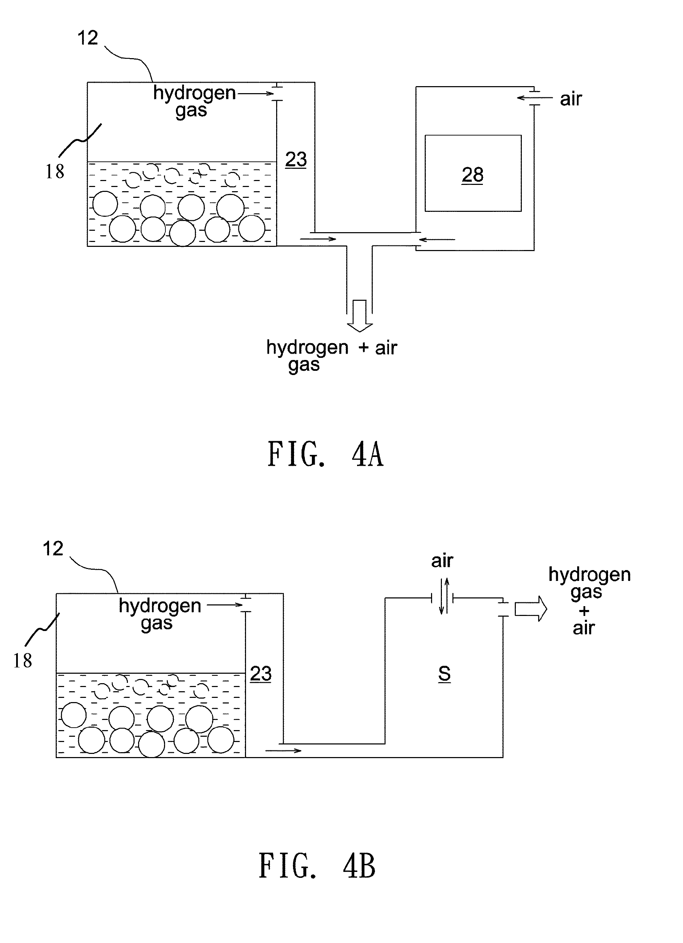

As shown in FIG. 3, a hydrogen production device 20 according to another embodiment includes a reaction unit 12, a hydrogen collection chamber 14, and a flow channel 18. In this embodiment, the hydrogen collection chamber 14 further includes an air-mixing region and an odor-adding region. Before the hydrogen gas flows into the hydrogen acceptor 16, the hydrogen gas may pass through the air-mixing region to reduce the concentration. In case the hydrogen acceptor 16 is a human body or an article (like a drink or cosmetics) used or taken by a person, the concentration of hydrogen gas flowing into the hydrogen acceptor 16 is preferably set as 0.1%-10% and particularly about 2%. Therefore, as shown in FIG. 4A, an air-transporting device 28 such as a fan or a pump is provided in the air-mixing region to mix air into the hydrogen gas so as to decrease the concentration of hydrogen gas. Alternatively, as shown in FIG. 4B, the air-mixing region may be formed by an air chamber S capable of exchanging air with the external environment, and the hydrogen gas from the reaction unit 12 is led into the air chamber S to adjust the concentration to fall in a proper range. Further, as shown in FIG. 3, the hydrogen gas may pass through an odor-adding region before entering the hydrogen acceptor 16 to add some odor. For example, the odor-adding region may be provided with a flavoring agent, a stabilizer, etc.

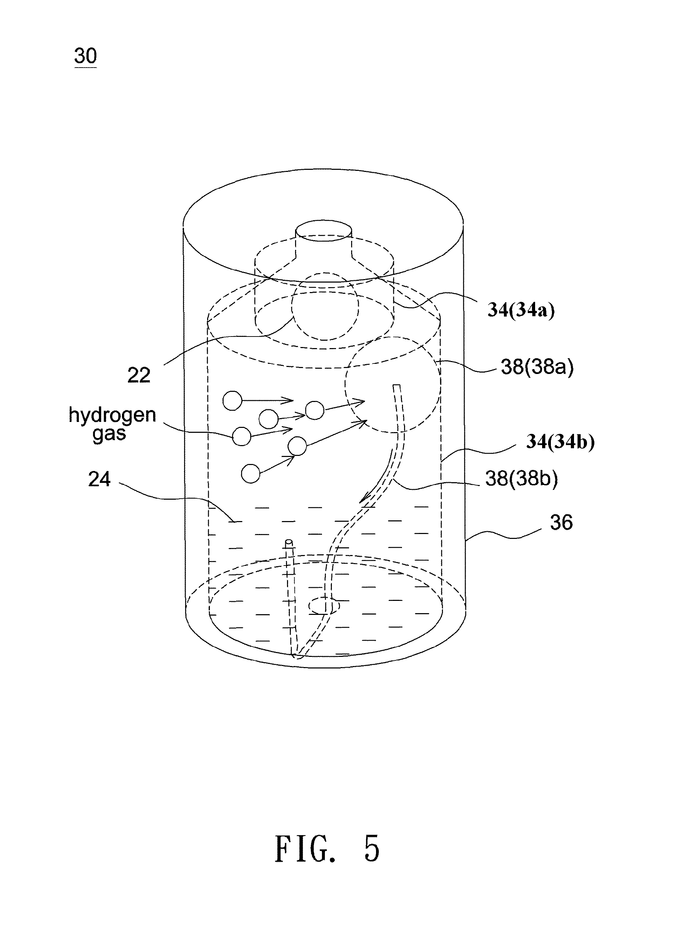

Referring to FIG. 5, a hydrogen production device 30 includes a reaction unit 34, a hydrogen collection chamber 36, and a flow channel 38. The reaction unit 34 has a first region 34a and a second region 34b. The first region 34a stores a first reactant 22, for example, a solid-state reactant, and the second region 34b stores a second reactant 24, for example, a liquid-state reactant. When the first region 34a is pressurized, the first reactant 22 enters the second region 34b and reacts with second reactant 24 to produce hydrogen gas in the reaction unit 34. Alternatively, the first reactant 22 may be a liquid-state reactant stored in the first region 34a, and the second reactant 24 may be a solid-state reactant stored in the second region 34b. The flow channel 38, for example, a liquid blocking flow channel, is in communication with the reaction unit 34 and the hydrogen collection chamber 36 to enable the hydrogen gas produced in the reaction unit 34 to flow into the hydrogen collection chamber 36. The flow channel 38 may include, for example, a water-repellent floating ball 38a and a water-repellent hydrogen conduit 38b. One end of the water-repellent hydrogen conduit 38b is connected to the water-repellent floating ball 38a, and the other end of the water-repellent hydrogen conduit 38b is connected to the hydrogen collection chamber 36. The water-repellent floating ball 38a is disposed inside the reaction unit 34. The hydrogen gas produced in the reaction unit 34 flows into the water-repellent floating ball 38a by a pressure difference, and the water-repellent floating ball 38a collects the produced hydrogen gas and then leads the hydrogen gas into the hydrogen collection chamber 36 through the water-repellent hydrogen conduit 38b. The water-repellent floating ball 38a may be made of any material having buoyancy, such as biodegradable material (made of corn), plastic, Styrofoam, permeable membrane, etc. Besides, the water-repellent floating ball 38a always expands with gas inside at various orientations to block the entrance of liquid.

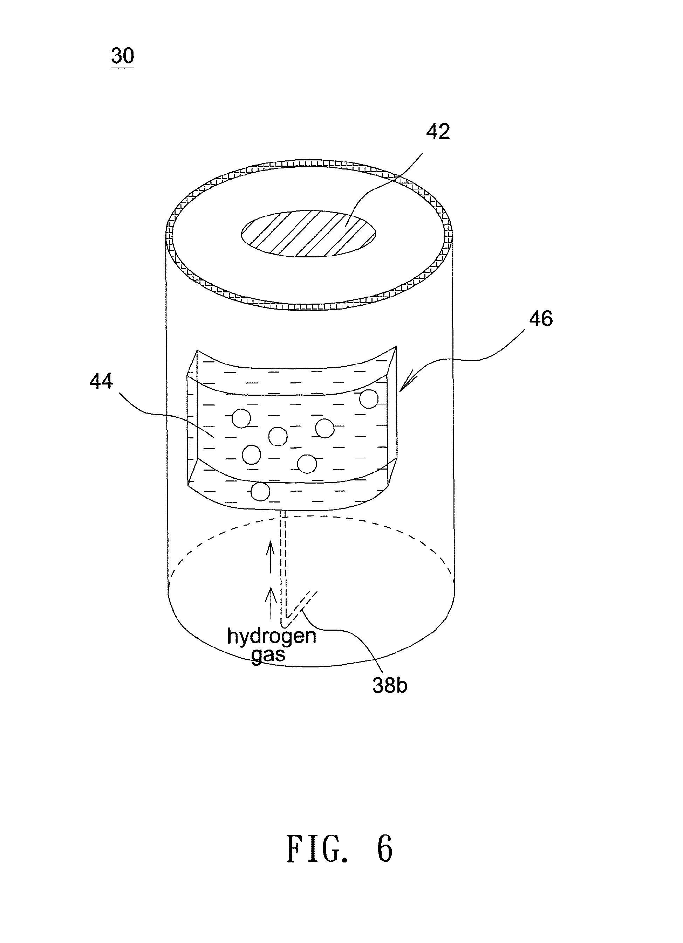

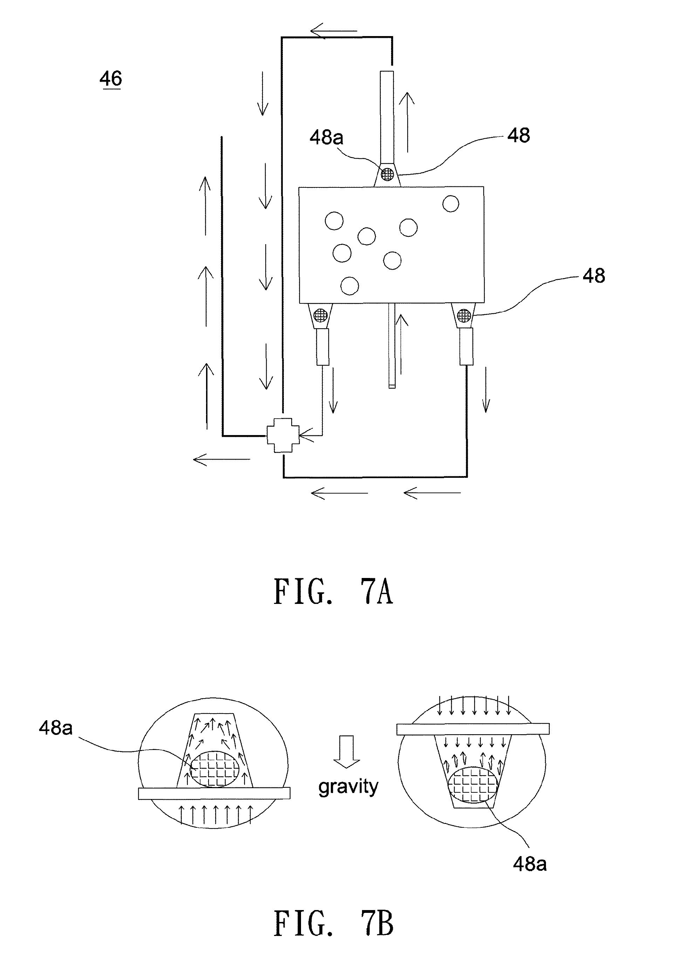

As shown in FIG. 6, a press-button region 42 is formed on the top of the hydrogen production device 30. When one presses the press-button region 42, the first reactant 22 (for example, a solid-state reactant) sinks down into the second reactant 24 (for example, a liquid-state reactant) or the first reactant 22 (for example, a liquid-state reactant) submerges the second reactant 24 (for example, a solid-state reactant) to react with each other. In one embodiment, a fluid region 44 may be formed in the hydrogen collection chamber 36. The hydrogen gas led out by the water-repellent hydrogen conduit 38b may be further led into the fluid region 44, and the hydrogen gas passing through the fluid region 44 and then leaving the hydrogen production device 30 forms bubbles in the fluid region 44. In that case, bubbles may be observed through an observation window 46 disposed on an outer wall of the hydrogen production device 30 to confirm whether the hydrogen gas is produced and to realize the reaction speed. Besides, the observation window 46 may also provide decorative effects. In addition, the fluid region 44 in the hydrogen collection chamber 36 may increase the humidity of hydrogen gas and precipitate impurities. Further, as shown in FIGS. 7A and 7B, the observation window 46 may include at least one check valve 48 (or non-return valve). The balls 48a in the check valve 48 block the passageway due to the force of gravity to prevent down-flowing liquid or gas from passing through the passageway. Accordingly, the hydrogen gas may flow along a preset direction indicated by arrows, and, even the hydrogen production device is rotated to a different orientation, the liquid in the observation window 46 does not overflow. Note the observation window 46 is not limited to be disposed on a specific position. For example, the observation window 46 may be disposed on other component or device described in each embodiment of the invention, depending on actual demands.

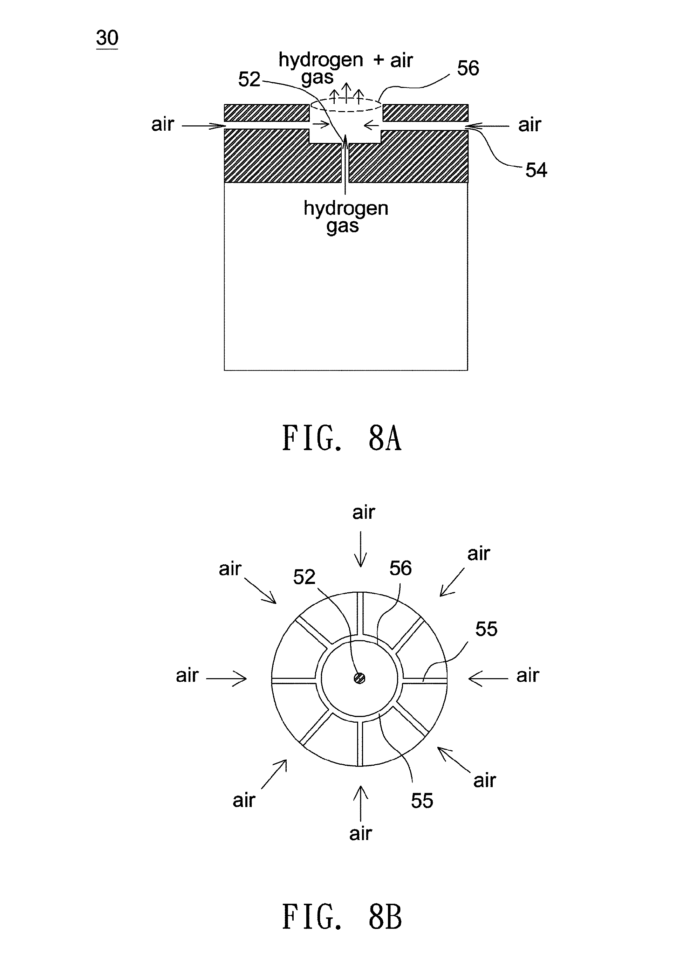

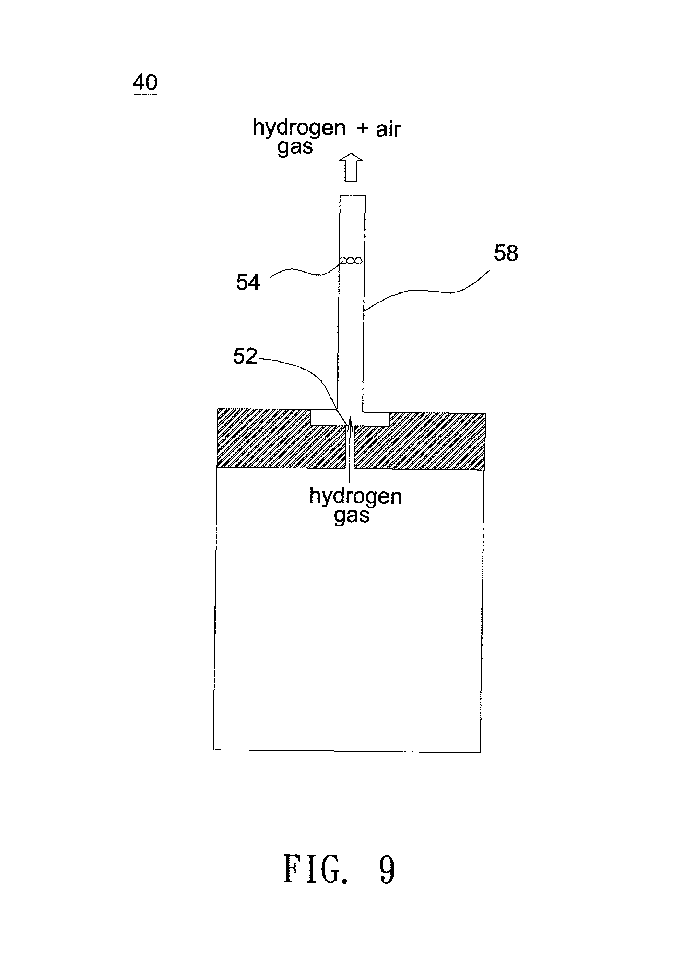

Referring to FIG. 8A, the hydrogen production device 30 includes a gas vent 52, and at least one through-hole 54 is formed on a side wall of the hydrogen production device 30 to form an air flow channel 55 in communication with the external environment. For example, as shown in FIG. 8A, when a person inhales hydrogen gas directly from the top of the hydrogen production device 30, a top suction force forms and draws outside air to the inside of the hydrogen production device 30 via the air flow channel 55. Under the circumstance, the hydrogen gas inhaled by a person via an air-mixing region 56 may have lower concentration. In one embodiment, as shown in FIG. 8B, in case a cross-sectional area of the gas vent 52 is designed to equal 2% of a cross-sectional area of the air-mixing region 56, the concentration of hydrogen gas inhaled by a person is about 2%. Certainly, the method for forming an air flow channel 55 is not limited. In an alternate embodiment shown in FIG. 9, a hydrogen production device 40 further includes a suction device 58 connected to the gas vent 52, and at least one through-hole 54 is formed on the suction device 58. When a person sucks hydrogen gas in the suction device 58, surrounding air flows into the suction device 58 via the through-hole 54 and mixes with the hydrogen gas in the suction device 58.

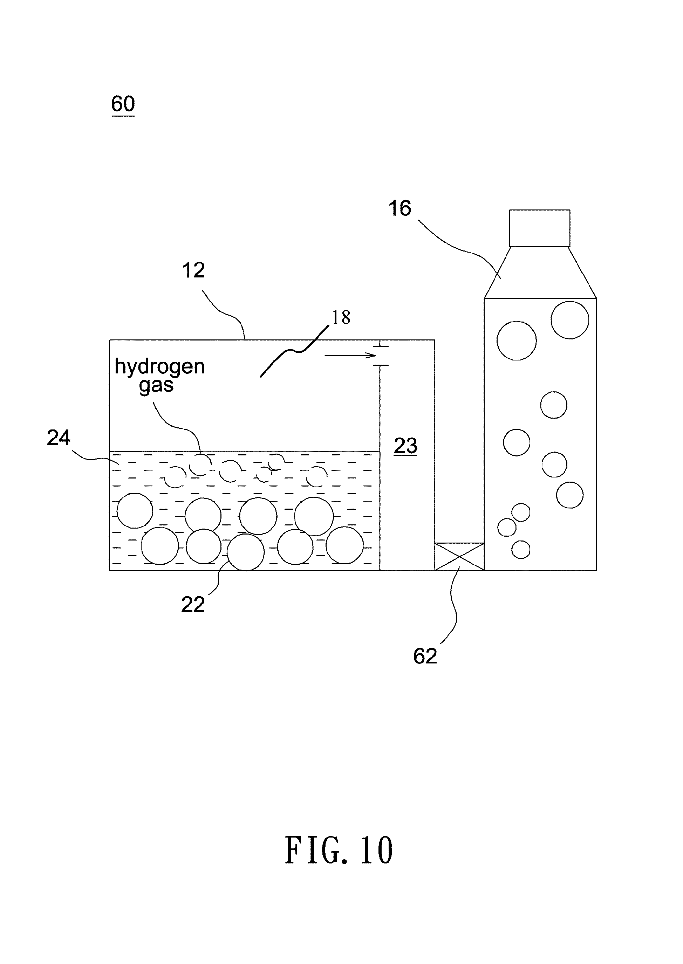

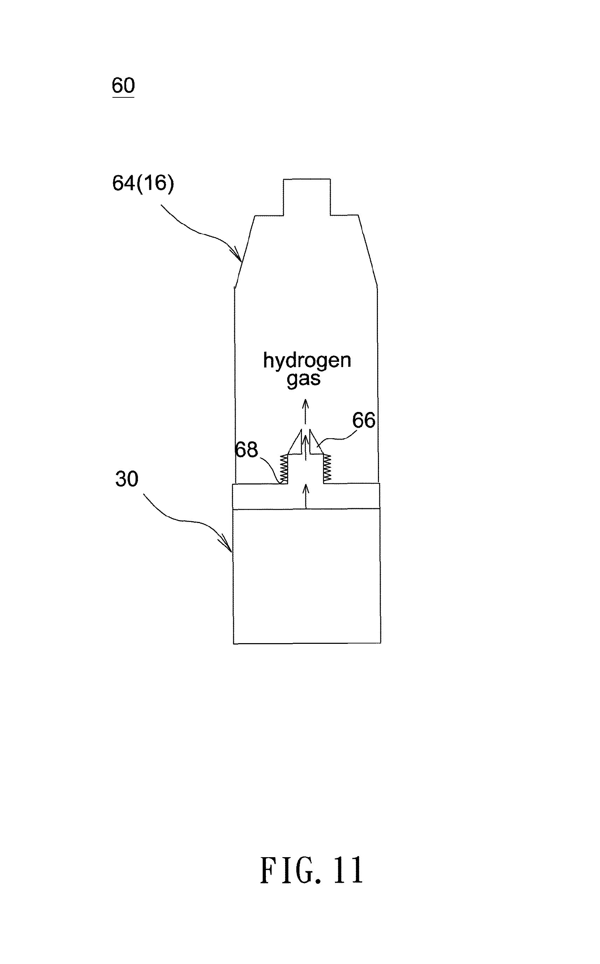

As shown in FIG. 10, a hydrogen addition device 60 includes a reaction unit 12, a flow channel 18, a penetration element 62, and a filtering region 23 formed between the reaction unit 12 and the penetration element 62. The reaction unit 12 includes a first reactant 22 and a second reactant 24, and the first reactant 22 comes into contact and reacts with the second reactant 24 to produce hydrogen gas. The penetration element 62 is disposed between the reaction unit 12 and a hydrogen acceptor 16. The hydrogen acceptor 16 may include, for example, a drink, cosmetics, etc. The penetration element 62 enables the reaction unit 12 and the hydrogen acceptor 16 to change from an isolation state to a communication state. For example, as shown in FIG. 11, in case the hydrogen acceptor 16 is a drink 64, the penetration element may be, for example, a helical ridge structure 66 with a sharp end. Besides, a thread structure 68 complementary to the helical ridge structure 66 in shape is formed on a side wall of the drink 64. The sharp end of the helical ridge structure 66 may pierce aluminum foils (a part of a side wall of the drink 64) or push away a plunger of the drink 64 to enable the hydrogen gas produced by the hydrogen production device 30 to flow into and slightly dissolve in the drink 64 immediately drunk by a person. Further, the helical ridge structure 66 is tightly fit with the thread structure 68 to avoid the leakage of liquid. In addition, the filtering region 23 formed between the reaction unit 12 and the penetration element 62 may purify hydrogen gas. Note the dimension of a hydrogen addition device could be arbitrarily given to conform to any practical situation. For example, the hydrogen addition device may have a dimension suitable to be carried and moved to realize a portable hydrogen addition device.

Based on the design of the hydrogen production device of the above embodiments, another embodiment of the invention provides a hydrogen-additive article. The hydrogen-additive article contains hydrogen gas supplied by a hydrogen production device. The hydrogen production device at least includes a reaction unit, a hydrogen collection chamber, and a flow channel. The flow channel is in communication with the reaction unit and the hydrogen collection chamber to enable a hydrogen gas produced in the reaction unit to flow into the hydrogen collection chamber. The hydrogen collection chamber has at least one gas vent to lead the hydrogen gas into the hydrogen-additive article. In this embodiment, the hydrogen-additive article includes, but not limited to, water, wine, beverage, and cosmetics.

In conclusion, the embodiment or the embodiments of the invention may have at least one of the following advantages.

According to the above embodiments, a person may directly inhale hydrogen gas just after the hydrogen gas is produced through a very simple operation, or the produced hydrogen gas is added into a hydrogen acceptor (such as a drink or cosmetics) and immediately taken or used by a person, with the concentration of the hydrogen gas being instantaneously adjusted in different circumstances. Accordingly, compared with conventional designs, the hydrogen production device according to the above embodiments is more portable, ready-to-serve, easy to add hydrogen gas into an article, and without the need of electronic devices or expensive components. Therefore, this also simplifies fabrication processes and lowers fabrication costs.

The foregoing description of the preferred embodiments of the invention has been presented for purposes of illustration and description. It is not intended to be exhaustive or to limit the invention to the precise form or to exemplary embodiments disclosed. Accordingly, the foregoing description should be regarded as illustrative rather than restrictive. Obviously, many modifications and variations will be apparent to practitioners skilled in this art. The embodiments are chosen and described in order to best explain the principles of the invention and its best mode practical application, thereby to enable persons skilled in the art to understand the invention for various embodiments and with various modifications as are suited to the particular use or implementation contemplated. It is intended that the scope of the invention be defined by the claims appended hereto and their equivalents in which all terms are meant in their broadest reasonable sense unless otherwise indicated. Therefore, the term "the invention", "the present invention" or the like does not necessarily limit the claim scope to a specific embodiment, and the reference to particularly preferred exemplary embodiments of the invention does not imply a limitation on the invention, and no such limitation is to be inferred. The invention is limited only by the spirit and scope of the appended claims. Moreover, these claims may refer to use "first", "second", etc. following with noun or element. Such terms should be understood as a nomenclature and should not be construed as giving the limitation on the number of the elements modified by such nomenclature unless specific number has been given. The abstract of the disclosure is provided to comply with the rules requiring an abstract, which will allow a searcher to quickly ascertain the subject matter of the technical disclosure of any patent issued from this disclosure. It is submitted with the understanding that it will not be used to interpret or limit the scope or meaning of the claims. Any advantages and benefits described may not apply to all embodiments of the invention. It should be appreciated that variations may be made in the embodiments described by persons skilled in the art without departing from the scope of the invention as defined by the following claims. Moreover, no element and component in the present disclosure is intended to be dedicated to the public regardless of whether the element or component is explicitly recited in the following claims.

## Claims

1. A hydrogen production device, comprising:
a reaction unit, storing a first reactant and a second reactant, wherein, when the first reactant comes into contact the second reactant, the first reactant reacts with the second reactant to produce hydrogen gas;
a hydrogen collection chamber having at least one gas vent to lead the hydrogen gas to flow into a hydrogen acceptor via the gas vent; and
a flow channel in communication with the reaction unit and the hydrogen collection chamber to enable the hydrogen gas to flow into the hydrogen collection chamber.

2. The hydrogen production device as claimed in claim 1, wherein one of the first reactant and the second reactant is a solid-state reactant and the other one is a liquid-state reactant.

3. The hydrogen production device as claimed in claim 1, wherein further comprises a barrier layer for separating the first reactant from the second reactant, wherein, when the barrier layer forms an opening, the first reactant comes into contact and reacts with the second to produce the hydrogen gas.

4. The hydrogen production device as claimed in claim 3, wherein the hydrogen collection chamber further comprises a filtering region to purify the hydrogen gas.

5. The hydrogen production device as claimed in claim 3, wherein the hydrogen collection chamber further comprises an odor-adding region.

6. The hydrogen production device as claimed in claim 1, wherein the flow channel comprises a water-repellent floating ball and a water-repellent hydrogen conduit, one end of the water-repellent hydrogen conduit is connected to the water-repellent floating ball, the other end of the water-repellent hydrogen conduit is connected to the hydrogen collection chamber, and the produced hydrogen gas flows into the water-repellent floating ball and the produced hydrogen conduit by a pressure difference.

7. The hydrogen production device as claimed in claim 1, wherein the hydrogen collection chamber comprises a fluid region.

8. The hydrogen production device as claimed in claim 1, further comprising:
an observation window used to confirm whether a hydrogen production reaction occurs.

9. The hydrogen production device as claimed in claim 8, wherein the observation window comprises at least one check valve.

10. The hydrogen production device as claimed in claim 1, further comprising:
at least one through-hole formed on a side wall of the hydrogen production device to form an air flow channel in communication with an external environment.

11. The hydrogen production device as claimed in claim 3 or 10, wherein the hydrogen collection chamber comprises an air-mixing region to adjust the concentration of the hydrogen gas flowing into the hydrogen acceptor.

12. The hydrogen production device as claimed in claim 11, wherein a concentration of hydrogen gas in the mixture is in a range of 0.1%-10% and a cross-sectional area of the gas vent is equal to about 2% of a cross-sectional area of the air-mixing region.

13. The hydrogen production device as claimed in claim 1, further comprising:
a suction device in communication with the gas vent, wherein at least one through-hole is formed on a side wall of the suction device to form an air flow channel in communication with an external environment.

14. The hydrogen production device as claimed in claim 1, wherein the hydrogen acceptor is a human body, a drink, or cosmetics.

15. A hydrogen addition device, comprising:
a reaction unit having a first reactant and a second reactant, wherein the first reactant comes into contact and reacts with the second reactant to produce hydrogen gas; and
a penetration element disposed between the reaction unit and a hydrogen acceptor, wherein the penetration element enables the reaction unit and the hydrogen acceptor to change from an isolation state to a communication state.

16. The hydrogen addition device as claimed in claim 15, wherein the hydrogen acceptor is a drink or cosmetics.

17. The hydrogen addition device as claimed in claim 15, wherein the penetration element penetrates or pushes a part of a side wall of the hydrogen acceptor to allow the hydrogen gas to dissolve in the hydrogen acceptor.

18. The hydrogen addition device as claimed in claim 15, wherein the penetration element has a helical ridge structure with a sharp end, the hydrogen acceptor has a thread structure complementary to the helical ridge structure in shape, the helical ridge structure is capable of piercing a part of a side wall of the hydrogen acceptor, and the helical ridge structure is tightly fit with the thread structure.

19. The hydrogen addition device as claimed in claim 15, further comprising a filtering region formed between the reaction unit and the penetration element to purify the hydrogen gas.

20. A hydrogen-additive article containing hydrogen gas supplied by a hydrogen production device, wherein the hydrogen production device at least comprises a reaction unit, a hydrogen collection chamber, and a liquid blocking flow channel, the flow channel is in communication with the reaction unit and the hydrogen collection chamber to enable a hydrogen gas produced in the reaction unit to flow into the hydrogen collection chamber, and the hydrogen collection chamber has at least one gas vent to lead the hydrogen gas into the hydrogen-additive article.
